# EUROPÄISCHE PATENTSCHRIFT

(11) **EP 1 885 314 B1**
(45) Veröffentlichungstag und Bekanntmachung des Hinweises auf die Patenterteilung: **27.07.2011**
(21) Anmeldenummer: 06753962.7
(22) Anmeldetag: 30.05.2006
(51) Int. Cl.: A61F 13/42, A61F 13/514

(54) **ABSORBIERENDER EINMAL-ARTIKEL**
ABSORBENT SINGLE-USE ARTICLE
ARTICLE JETABLE ABSORBANT

(30) Priorität: 03.06.2005 DE 102005026634
(43) Veröffentlichungstag der Anmeldung: 13.02.2008
(73) Patentinhaber: Paul Hartmann AG, 89522 Heidenheim (DE)
(72) Erfinder: SWEREV, Maximilian, 86169 Augsburg (DE); BODMER, Magnus, 89231 Neu-Ulm (DE); KESSELMEIR, Rüdiger, 89542 Herbrechtingen (DE); HORNUNG, Fridmann, Penalolén, SANTIAGO (CL)
(74) Vertreter: Friz, Oliver
(86) Internationale Anmeldenummer: PCT/EP2006/005126
(87) Internationale Veröffentlichungsnummer: WO 2006/128665

(56) Entgegenhaltungen:
- EP-A1- 0 211 524
- EP-A1- 0 553 808
- EP-A2- 0 813 850
- WO-A-01/41691
- WO-A-99/16401
- US-A- 4 834 733
- US-A- 5 035 691
- US-A1- 2003 154 904
- US-A1- 2004 064 113

## Beschreibung

Die Erfindung betrifft einen absorbierenden Einmal-Artikel umfassend einen Körperflüssigkeiten speichernden Saugkörper und ein zumindest abschnittsweise flüssigkeitsundurchlässiges Rückenblatt, wobei das flüssigkeitsundurchlässige Rückenblatt eine mikroporöse atmungsaktive Folie aufweist und wobei auf der dem Saugkörper zugewandten Seite der Folie ein Nässeindikator vorgesehen ist.

US-A-2004/064113 A1 offenbart einen solchen absorbierenden Einmalartikel, wobei eine mikroporöse atmungsaktive Folie nur als eine von mehreren Möglichkeiten der Gestaltung des Backsheets erwähnt ist. Daneben werden in dieser Druckschrift eine Vielzahl von Nässeindikatormaterialien und deren Aufbringung und Anordnung in verschiedenster Weise innerhalb des Einmalartikels, insbesondere auf zusätzlichen Trägerschichten oder auch direkt auf der Innenseite des Rückenblatts, angesprochen.

Es bestand schon lange das Bedürfnis, eine Einnässung eines absorbierenden Einmal-Artikels durch geeignete Mittel anzuzeigen. Hierfür sind verschiedene Nässeindikatoren bekannt geworden. Die Nässeindikatoren sind überwiegend im Bereich des Saugkörpers und auf der körperabgewandten Seite des Saugkörpers vorgesehen, um eine Einnässung des Saugkörpers anzuzeigen.

So sind Nässeindikatoren auf Basis von pH-Indikatoren bekannt, die bei Einnässung mit Körperflüssigkeiten, je nach pH-Wert, einen Farbumschlag zeigen und dadurch eine Einnässung anzeigen. Eine solche Verfärbung ist häufig in Bezug auf die schwache Kontrastierung, beispielsweise von Gelb nach schwach Blau, nur schwer wahrzunehmen. Diese pH-Indikatoren können beispielsweise als Bestandteil einer Hotmeltzusammensetzung aufgebracht werden (US 5,035,691). Die Herstellung von Hotmelt-Nässeindikatoren und deren Aufbringung, zumeist durch Schlitzkopfauftrag, ist aufwendig.

Daneben sind Nässeindikatoren auf Basis wasserlöslicher Tinten bekannt und etwa in EP 0 211 524 B1 beschrieben. Derartige Nässeindikatoren sollen sich bei Kontakt mit Körperflüssigkeiten lösen und verteilen. Sie können beispielsweise durch Druckwerke, insbesondere durch Tintenstrahlbedruckung, auf entsprechende Komponenten des Einmal-Artikels, insbesondere auf dessen Rückenblatt aufgebracht werden. So beschreibt EP 0 211 524 B1 die Beaufschlagung einer undurchlässigen Plastikfolie als Rückenblatt mit wasserlöslichem Farbstoff zum Beispiel in Form von Graphiken, welche bei Einwirkung von Körperflüssigkeit sich verändern, insbesondere verwischen.

Wenn Nässeindikatoren der zuletzt genannten Art bei Einmal-Artikeln mit mikroporösen atmungsaktiven Folien als Rückenblatt (Backsheet) verwendet werden, so ergeben sich einerseits Probleme mit der Sichtbarkeit. Die mikroporösen Folien sind nämlich im Allgemeinen relativ undurchsichtig, da durch die luftgefüllten Poren verschiedene Änderungen des Brechungsindexes zu hoher Opazität (Milchigkeit) führen. Es ist diesbezüglich aber bereits vorgeschlagen worden, die Folien auf geeignete Weise thermisch und/oder mechanisch nachzubehandeln und hierdurch eine Verringerung der Opazität, d. h. eine bessere Durchsichtbarkeit (Transparenz) zu erreichen (EP 0 553 808 B1).

Andererseits stellt sich ein grundsätzliches Problem des Funktionierens der Nässeanzeige bei der Verwendung solcher Nässeindikatoren auf Basis wasserlöslicher Systeme, wie wasserlöslicher Tinten, in Verbindung mit mikroporösen atmungsaktiven Folienmaterialien. Aus bislang nicht untersuchten Gründen waren keine zufriedenstellenden Nässeindikatoren im Zusammenwirken mit mikroporösen, atmungsaktiven Folien bekannt. Es wurde zwar vorgeschlagen, den Nässeindikator auf oder benachbart zu einem Streifen, separat von dem Backsheet, aufzubringen, welcher Streifen sich farblich von dem übrigen Backsheetmaterial unterscheidet, so dass für Pflegepersonal der Ort der Anbringung des Nässeindikators leichter identifizierbar ist. Damit wurde aber das grundsätzliche Problem des nur unbefriedigend funktionierenden Nässeindikators auf Basis wasserlöslicher Tinten nicht gelöst

Auch gemäß EP 0 813 850 A2 wird dieses grundsätzliche Problem des unzureichenden Funktionierens von solchen Nässeindikatoren bei mikroporösen Backsheets nicht gelöst, sondern der Nässeindikator ist auf eine zusätzliche von dem Backsheet separate Schicht aufgebracht, was wiederum den Herstellungsaufwand erhöht und die Kontrastierung herabsetzt und die Erkennbarkeit beeinträchtigt.

Es ist daher Aufgabe der vorliegenden Erfindung, ein zufriedenstellend funktionierendes, einfaches und kostengünstiges Nässeindikatorsystem für absorbierende Einmal-Artikel mit atmungsaktivem Rückenblatt zur Verfügung zu stellen.

Diese Aufgabe wird gelöst durch die Bereitstellung eines absorbierenden Einmal-Artikels mit den Merkmalen des Anspruches 1.

Dadurch, dass der Nässeindikator visuell wahrnehmbar in Form einer strukturierten Anordnung direkt auf die mikroporöse atmungsaktive Folie aufgebracht ist und sich von dieser Folie bei Kontakt mit wässriger Flüssigkeit bis zur Unkenntlichkeit ablöst, ist jederzeit ein sicherer Hinweis auf die erfolgte Einnässung des Einmal-Artikels gegeben.

Die Ablösung der visuell wahrnehmbaren strukturierten Anordnung des Nässeindikators von der Folie erfolgt bei bestimmungsgemäßem Gebrauch des Produktes, zum Beispiel bei entsprechender Einnässung einer Windel. Die Ablösung der visuell wahrnehmbaren strukturierten Anordnung des Nässeindikators von der Folie kann insbesondere aber auch durch den nachfolgend erläuterten Abwisch-Test ermittelt werden.

### Abwischtest:

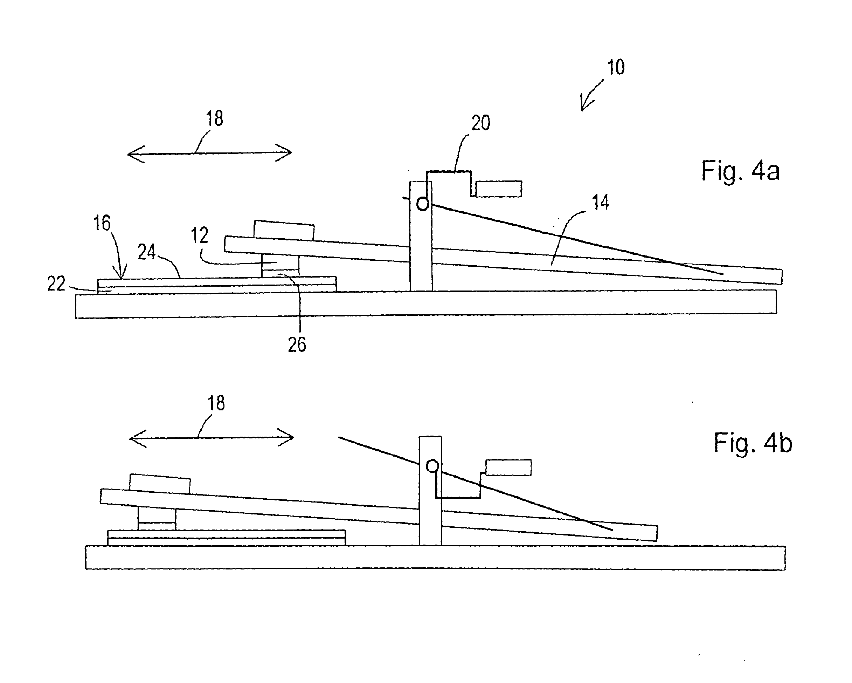

Dieser Test ist angelehnt an die europäische Norm EN ISO 105-X16:2002(D) (Farbechtheitsprüfungen), welche Norm die Widerstandsfähigkeit von Farben bei Textilien gegen Abreiben bestimmen soll. Zur Durchführung des Abwischtests wird das nach 4.1 der Norm normkonforme Gerät verwendet, welches in Figur 4 schematisch dargestellt ist. Figur 4 zeigt das Prüfgerät 10, mittels dessen ein unter vorgegebener Normalkraft von 11,1 +/- 0,5 N stehender zylindrischer Prüfzapfen 12 mit einem Durchmesser von 16 +/- 0,1 mm Durchmesser mittels einer im Wesentlichen horizontal verlaufenden Schubstange 14 horizontal auf einem Prüfling 16 hin und her gezogen wird. Diese Hin- und Herbewegung in Richtung des Doppelpfeils 18 wird durch einen Kurbelmechanismus 20 erzeugt. Der Prüfling 16 ist gebildet aus einem das Rückenblatt 22 bildenden mikroporösen atmungsaktiven Folienmaterial, auf dessen Oberfläche unmittelbar und direkt der Nässeindikator 24 aufgebracht wurde, insbesondere aufgedruckt wurde und unter Verdampfung des Lösungsmittels getrocknet wurde. An der dem Prüfling 16 zugewandten Seite des Prüfzapfens 12 ist ein Standardbaumwollgewebe 26 in Form eines Begleitgewebes aus Baumwolle gemäß ISO 105-F: 1985 vorgesehen (erhältlich von Firma Testex Prüftextilien, Bad Münstereifel, DE). Dieses Standardbaumwollgewebe wurde mit demineralisiertem Wasser getränkt, derart dass eine Wasseraufnahme von 95% bis 100% nach 6.3 der Norm vorliegt.

Für die Durchführung des Abwischtests wird über den Kurbelmechanismus 20 eine Hin- und Herbewegung in Richtung des Doppelpfeils 18 ausgeführt, und es wird dann das Resultat in Augenschein genommen. Wenn der zuvor aufgebrachte Nässeindikator 24 bis zur Unkenntlichkeit verwischt oder abgewischt ist, so eignet sich das System aus Nässeindikator 24 und Rückenblatt 22 für die beabsichtigten Zwecke.

Der Nässeindikator ist insbesondere dann geeignet, wenn der Nässeindikator nach dem beschriebenen Wischtest in einer höchstens 20-maligen Hin- und Herbewegung des Prüfzapfens 12, vorzugsweise in einer höchstens 10-maligen Hin- und Herbewegung, besonders bevorzugt in einer höchstens fünfmaligen Hin- und Herbewegung bis zur Unkenntlichkeit abgewischt ist.

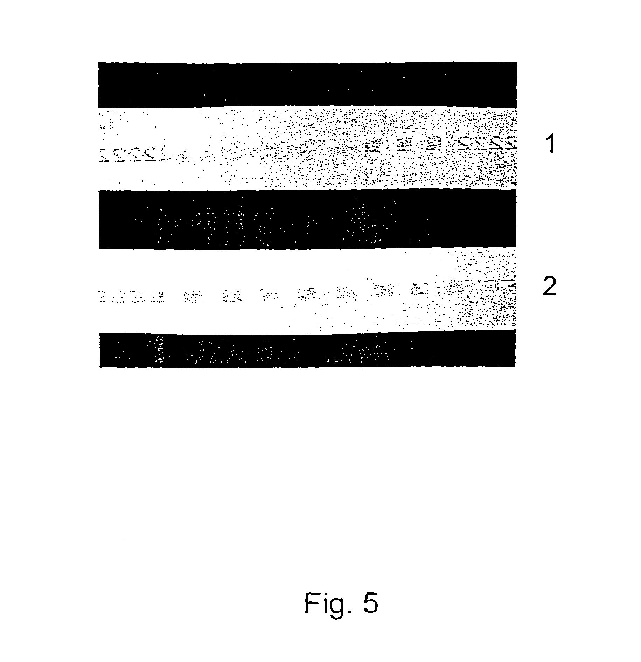

Beispielsweise zeigt Figur 5 das Ergebnis beim beschriebenen Wischtest bei einem erfindungsgemäßen System 1 (oben) und bei einem nicht erfindungsgemäßen System 2 (unten). Bei dem erfindungsgemäßen System 1 ist die strukturierte Anordnung des Nässeindikators nach fünfmaliger Hin- und Herbewegung innerhalb des Bereichs der Hin- und Herbewegung bis zur Unkenntlichkeit verwischt. Bei dem nicht erfindungsgemäßen System 2 ist sogar nach 50-maligem Hin- und Herbewegen des Prüfzapfens 12 die strukturierte Anordnung des Nässeindikators nicht bis zur Unkenntlichkeit verwischt, sondern noch visuell vollständig erkennbar. Bei dem Nässeindikator in System 1 handelt es sich um eine in der Uhrenindustrie verwendete Tinte mit einem Azofarbstoff und Polyvinylpyrrolidon als Bindepolymer (erhältlich als Typ 5533, der Firma Imaje, Stuttgart, DE). Das mikroporöse atmungsaktive Rückenblatt (Typ Hypor B 140, von der Firma RKW, Wasserburg, DE) ist ein Vlies/Folienlaminat bestehend aus einer mikroporösen atmungsaktiven Folie und einem Spinnvlies. Das Flächengewicht der blasextrudierten Polypropylen-Folie beträgt 20,5 g/m². Die in Maschinenrichtung gereckte Folie weist einen Kreideanteil (CaCO₃) als die Mikroporosität bewirkenden Bestandteil von 50 bis 60 Gew.-% bezogen auf die Masse der Folie auf. Das Flächengewicht des thermisch vollflächig verbundenen Laminats beträgt 35 g/m², wobei das Spinnvlies auf die dem Nässeindikator abgewandte Seite der Folie aufgebracht ist. Bei dem Nässeindikator-System (2) unten handelt es sich um den Standardnässeindikator FT 123 von der Firma Imaje mit einem Triphenylmethan-Farbstoff, wobei das Rückenblatt dasselbe wie bei dem Nässeindikator-System 1 (oben) ist. Wechselwirkungen zwischen dem Nässeindikator und der mikroporösen atmungsaktiven Folie sind jedenfalls derart, dass auch nach 50-maligem Hin- und Herbewegen des Druckkörpers die strukturierte Anordnung des Nässeindikators fast unverändert erkennbar blieb, obschon der Standardnässeindikator Image FT 123 als wasserlöslich zu bezeichnen ist.

Auch wenn die Funktionsweise erfindungsgemäßer Nässindikatorsysteme noch nicht erschöpfend geklärt ist, so wurde vorliegend doch festgestellt, dass ein zufriedenstellendes Ansprechen des Nässeindikators bei Einnässung nicht nur auf die besonderen chemischen und physikalischen Eigenschaften der mikroporösen atmungsaktiven Folienmaterialien und auf die chemischen Wechselwirkungen zwischen den Folienmaterialien, die zumeist Polyethylen und/oder Polypropylen umfassen, und dem Nässeindikator zurückzuführen sind, sondern es müssen auch Wechselwirkungen mit den anorganischen Füllstoffen, im Allgemeinen CaCO₃, sowie mögliche geometrische Effekte zwischen der Oberflächenstruktur und/oder Porenstruktur der mikroporösen atmungsaktiven Folie und dem Nässeindikator berücksichtigt werden. Dies ist seither nicht erkannt worden.

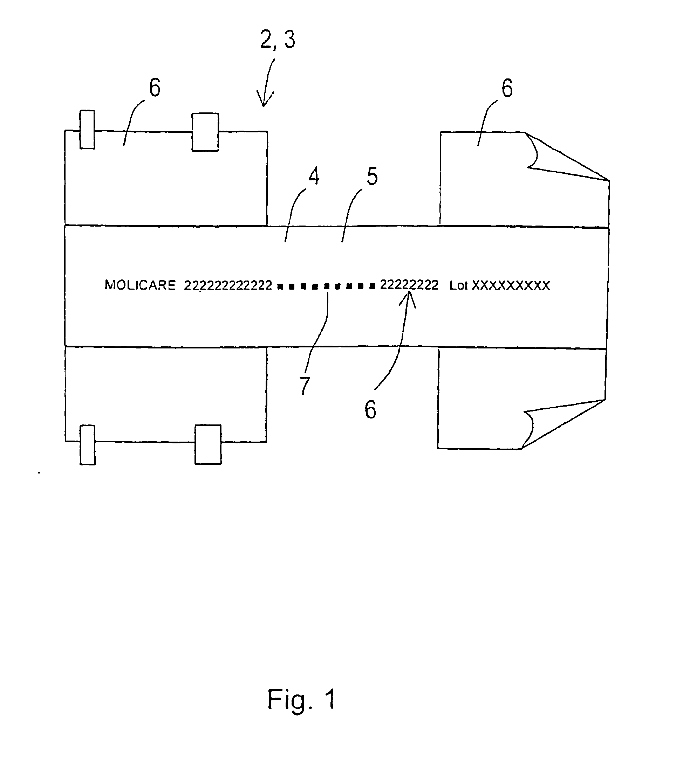

Durch eine systematische Betrachtung der möglichen Wechselwirkungen und demgemäß durch eine gezielte Auswahl verschiedener mikroporöser atmungsaktiver Folienmaterialien als Rückenblattkomponente (Backsheet) eines Einmal-Artikels in Kombination mit geeigneten tintenbasierten Nässeindikator-Systemen wird erstmals die Möglichkeit der Schaffung eines funktionierenden Indikatorsystems auf dieser Basis geschaffen. Dabei ist es nicht notwendig, das Folienmaterial in dem für den Nässeindikator vorgesehenen Bereich durch zusätzliche Beschichtungen zu behandeln bzw. den Nässeindikator auf separate Schichtstoffe aufzubringen. Die direkte Aufbringung des Nässeindikators auf die mikroporöse atmungsaktive Folie des Rückenblattes in einer entsprechenden visuell wahrnehmbaren Schichtdicke und strukturierten Anordnung und Kontrastierung gewährleistet die Wahrnehmung des Nässeindikators auch bei mikroporöses atmungsaktives Folienmaterial aufweisenden Rückenblättern. Der Nässeindikator (7) ist in Form einer visuell wahrnehmbaren, direkt auf die Folie aufgebrachten strukturierten Anordnung(6) vorgesehen, wie schematisch in Figur 1 dargestellt, wobei sich die strukturierte Anordnung bei Kontakt mit wässriger Flüssigkeit bis zur Unkenntlichkeit ablöst. Es kann hierdurch eine gute Sichtbarkeit der Indikation des Nässeindikators erzielt werden, was sich insbesondere für ältere Personen mit verringerter Sehfähigkeit als vorteilhaft erweist, aber auch für Pflegepersonal, insbesondere bei Pflegemaßnahmen während der Nacht, von Vorteil ist.

Unter absorbierenden Einmal-Artikeln werden alle zum einmaligen Gebrauch vorgesehenen Artikel verstanden, deren Funktion in der Aufnahme von Körperflüssigkeiten wie beispielsweise Urin, dünnflüssigem Stuhl, Blut oder Wundexudat liegt.

Absorbierende Einmal-Artikel sind insbesondere Windeln und Vorlagen für Babys, Kleinkinder und inkontinente Erwachsene.

In einem weiteren Erfindungsgedanken werden unter absorbierenden Einmal-Artikeln ebenso absorbierende Abdecktücher beispielsweise für den OP-Bereich oder auch Wundauflagen verstanden.

Als flüssigkeitsundurchlässiges Rückenblatt wird die Lage oder der Lagenverbund verstanden, die auf der dem Körper abgewandten Seite des absorbierenden Einmal-Artikels eine Barrierefunktion für die Körperflüssigkeiten erfüllt.

Nach einer bevorzugten Ausführungsform ist die strukturierte Anordnung des Nässeindikators in Form von visuell identifizierbaren Symbolen, Codierungen, Ziffern, Schriftzeichen oder sonstigen abstrakten Graphiken aufgebracht.
Weiter vorteilhafterweise gibt die strukturierte Anordnung des Nässeindikators Informationen über den Einmal-Artikel wieder, im Sinne von beispielsweise Produktname, Lot-Nummer, Produktgröße oder Saugkapazität.

Vorzugsweise ist die strukturierte Anordnung blockweise gestaltet. Auf diese Weise kann sofort und ohne weiteres festgestellt werden, wenn sich die strukturierte Anordnung bei Kontakt mit wässriger Flüssigkeit ablöst oder auflöst.

Besonders bevorzugt ist die strukturierte Anordnung des Nässeindikators auf der Folie in Längsrichtung des Einmal-Artikels orientiert. Ganz besonders bevorzugt ist der Nässeindikator auf der Folie derart aufgebracht, dass im an eine Person angelegten Zustand des absorbierenden Einmal-Artikels der Nässeindikator sich ausgehend von dem vorderen Bereich des absorbierenden Einmal-Artikels über den Schrittbereich bis zu dem hinteren Bereich des Einmal-Artikels erstreckt. Vorteilhafterweise erstreckt sich der Nässeindikator in seiner strukturierten Anordnung auf der Folie über die Länge des im Einmal-Artikel vorgesehenen Saugkörpers. Vorteilhafterweise ist der Nässeindikator auf der Folie innerhalb eines Bereiches vorgesehen, der dem Bereich des Saugkörpers entspricht.

Die strukturierte Anordnung des Nässeindikators ist über entsprechende Kontrastierung durch das Rückenblatt hindurch auf der dem Saugkörper abgewandten Seite visuell erkennbar. Die Zusammensetzung des Nässeindikators enthält mindestens ein Farbmittel. Das Farbmittel ist entnommen aus der Gruppe der Farbstoffe und Pigmente.
Vorzugsweise umfasst der Nässeindikator einen wasserdispergierbaren bzw. wasserablösbaren und/oder wasserlöslichen Farbstoff.

Zudem erweist es sich als vorteilhaft, wenn der Nässeindikator mit einer Schichtdicke von wenigstens 2 µm, insbesondere von wenigstens 5 µm und weiter insbesondere von 10 bis 25 µm auf das Rückenblatt aufgebracht ist. Der Auftrag des Nässeindikators in Form des Trockengewichtes beträgt vorteilhafterweise wenigstens 0,00024 g/cm², insbesondere wenigstens 0,00049 g/cm² und weiter insbesondere wenigstens 0,0024 g/cm².

Die vorausgehend erwähnten Wechselwirkungen lassen sich durch nachfolgend zu erörternde weitere Testmaßnahmen noch weitergehend erfassen; wesentlich sind hierbei die Wechselwirkung zwischen visuell wahrnehmbaren Bestandteilen des Nässeindikators, insbesondere der Farbstoffe, mit der Oberfläche des mikroporösen Rückenblatts und dessen Bestandteilen, also insbesondere den anorganischen Füllstoffen, durch welche in an sich bekannter Weise durch Recken des Folienmaterials eine Mikroporosität der Folie hergestellt wird, indem die zuvor fest verankerten Füllstoffe in dem Folienverbund gelockert werden, wodurch in bekannter Weise die Mikroporosität erzeugt wird. Es wurde festgestellt, dass der Polarität der Bestandteile des Nässeindikators einerseits und der Polarität des Rückenblatts bzw. seiner Bestandteile eine entscheidende maßgebliche Bedeutung im Hinblick auf das Funktionieren des Nässeindikators zukommt. Mikroporöse Folien haben meist durch Zugabe der CaCO₃-Partikel polare Oberflächen. Besteht diesbezüglich eine hohe Affinität des Nässeindikators zu der Unterlage, so löst sich der Nässeindikator auch bei Einnässung nicht in zufriedenstellender Weise ab, obschon an sich eine Wasserlöslichkeit bzw. Dispergierbarkeit in wässrigen Flüssigkeiten der maßgeblichen Bestandteile gegeben ist.

Bei üblicherweise verwendeten Tinten als Nässeindikator-Substanz handelt es sich um dünnflüssige Zusammensetzungen auf Wasser- und Lösungsmittel-Basis mit einer Viskosität von 4 bis 30 mPA.s, oftmals bestehend aus einem Gemisch organischer Lösungsmittel (zum Teil bis zu acht Komponenten zur Steuerung der Trocknungszeit) und umfassend Farbmittel. Als Lösungsmittel werden vorzugsweise Ketone (vorzugsweise Methylethylketon), Acetate (Ester), Glykolether und Alkohole (z.B. Ethanol)eingesetzt. Der Lösungsmittel-Anteil beträgt in der Regel 80 bis 90 %(m/m). Die Farbmittel (3 bis 4 %(m/m)) sind vorwiegend lösliche Farbstoffe mit hoher Lichtechtheit und Thermostabilität, zum Teil Pigmente mit einer Teilchengröße unter 3 µm. Als Bindemittel enthalten die Tinten 5 bis 15% Kunstharze aus einem oder mehreren Polymertypen zur Kontrolle von Viskosität, Tropfenbildung und Bindung der Farbmittel an die zu bedruckende Oberfläche. Mit Additiven (weniger als 1 %(m/m)) lassen sich u. a. die Fließeigenschaften beeinflussen, um einen definierten Tropfenstrom zu gewährleisten, sie wirken auch als Weichmacher im Bindemittel. Vorzugsweise wird darin auch ein leitfähiges Salz zur Erzielung einer elektrischen Leitfähigkeit von > 10⁵ Ω⁻¹ cm⁻¹ verwendet, damit die Tintentröpfchen zur Bildung eines Schriftbildes im elektrischen Feld abgelenkt werden können.

Um die Funktion des Nässeindikators zu gewährleisten, wurde festgestellt, dass die Polarität des verwendeten Farbstoffs in Kombination mit geeigneten Polymertypen im Nässeindikator, die als Bindemittel die Haftung zum Untergrund, also dem mikroporösen atmungsaktiven Folienmaterial, herstellen, optimal auf die Eigenschaften dieses mikroporösen, atmungsaktiven Folienmaterials des Rückenblatts abgestimmt werden können. Ein sehr gutes Werkzeug zur Beurteilung der Polarität von Molekülen ist die Dünnschichtchromatographie (DC) in Form der Normalphasendünnschichtchromatographie. Hier wird die Trennung eines Stoffs auf einer stationären Phase mit bestimmter Polarität in Wechselwirkung mit einer mobilen Phase bestimmter Polarität durchgeführt: So kann eine Trennung auf einer polaren stationären Phase mit einem unpolareren Lösungsmittel oder Lösungsmittelgemisch einer definierten Eluotropie vorgenommen werden. Aus der zurückgelegten Laufstrecke einer zu analysierenden Verbindung oder eines zu analysierenden Stoffs im Verhältnis zur Laufstrecke der mobilen Phase, also der eigentlichen Trennstrecke, wird der sogenannte R_{F}-Wert ermittelt. Der R_{F}-Wert ist dabei ein Maß für die Stärke der Wechselwirkung des Stoffes oder Verbindung mit der stationären Phase. Je stärker die Wechselwirkung mit dem Untergrund, also der stationären Phase ist, umso weniger weit erfolgt der Transport mit dem Laufmittel auf der stationären Phase. Mit dem DC-System einer polaren stationären Phase mit einer unpolareren mobilen Phase können Farbstoffe als Nässeindikator ausgewählt werden, die die erforderliche geringe Wechselwirkung mit den polaren Funktionen der atmungsaktiven Folie aufweisen. Wie sich in den vergleichenden Untersuchungen verschiedener Tinten unterschiedlicher Hersteller gezeigt hat, können hier prinzipiell Tintenfarbstoffe mit schwacher polarer Wechselwirkung gegenüber Tinten mit deutlich stärkerer Wechselwirkung voneinander unterschieden werden.

Es erweist sich in besonderem Maße als vorteilhaft, wenn ein Nässeindikator auf einer polaren stationären Phase in Form einer Dünnschichtplatte gemäß der nachfolgend noch näher beschriebenen Testmethode der Dünnschichtchromatographie einen R_{F}-Wert größer 0,48 aufweist.

Viele der üblicherweise bekannten Tinten basieren auf Triphenylmethan-Farbstoffen, die alle einen R_{F}-Wert ≤ 0,48 aufweisen und sich auf polaren Oberflächen von mikroporösen atmungsaktiven Folienmaterialien nicht oder nicht ausreichend ablösen lassen. Wird hingegen ein Nässeindikator mit einem R_{F}-Wert größer als 4,8 verwendet, beispielsweise aus der Gruppe der Azofarbstoffe, so bedeutet dies, dass die Wechselwirkung zwischen Nässeindikator bzw. Farbstoff des Nässeindikators und dem polaren Untergrund nicht derart ist, dass ein Ablösen oder Auflösen des Nässeindikators bei Flüssigkeitsbeaufschlagung behindert wird, so dass der Nässeindikator visuell wahrnehmbar funktioniert.

Mit Hilfe der Dünnschichtchromatographie können somit Nässeindikatoren bzw. die dafür eingebrachten Farbstoffe ausgewählt werden, die sich grundsätzlich für diesen Einsatzzweck bei zumeist polaren mikroporösen atmungsaktiven Folienmaterialien als Rückenblatt eines absorbierenden Einmal-Artikels eignen.

Der vorstehend erwähnte Test der Dünnschichtchromatographie wird wie folgt durchgeführt:
Zur Durchführung einer Dünnschichtchromatographie wird als stationäre Phase ein plattenartiger Träger 20 x 20 cm aus Polyester-Folie und einer auf die Polyester-Folie aufgebrachten Schicht aus Kieselgel 60 (Kieselgel mit einer mittleren Porengröße von 60 Å = 6 nm)verwendet. Die Schicht aus Kieselgel hat eine spezifische Oberfläche von ca. 500 m²/g und eine Körnung von 5 bis 17 µm. Ihr ist ein Fluoreszenz-Indikator zugesetzt, der unter UV-Licht von 254 nm fluoresziert, sowie Gips als Bindemittel. Die Schichtdicke der Kieselgel-Schicht beträgt 0,25 mm. Eine so beschaffene stationäre Phase ist als "DC-Fertigfolie Polygramm SIL G/UV254" erhältlich von der Firma Macherey-Nagel GmbH & Co. KG, Düren, DE.

Auf diese stationäre Phase wird mit einer Bandbreite von 5 mm und unter Verwendung einer sehr exakten Auftragvorrichtung jeweils 5 µl einer oder mehrerer Prüflösungen zu Beginn einer Trennstrecke von 7 cm Länge aufgebracht. Hierfür kann eine Auftragvorrichtung der Marke Linomat III von der Firma CAMAG, Berlin, DE verwendet werden. Zur Herstellung der Prüflösungen wird jeweils eine Menge von 200 mg einer Farbstoffzubereitung mit Ethanol 96% (vergällt mit 1% Methylethylketon) oder einem anderen geeigneten Lösemittel auf ein Volumen von 20 ml verdünnt. Die DC-Platte wird nach Auftrag der Prüflösungen trocknen gelassen.

Die so vorbereitete stationäre Phase wird aufrecht in eine mit der mobilen Phase aus unpolareren Lösungsmittel oder Lösungsmittelgemisch befüllte und kammergesättigte Doppeltrogkammer mit Deckel eingesetzt, um ein aufsteigendes Trennverfahren mit Kammersättigung durchzuführen. Die mobile Phase(synonym mit Laufmittel) umfasst (bei 20°C) folgende Reagenzien/Zusammensetzungen, die in Volumenanteilen von 50:10:10:10 vorliegen:
- n Butanol zur Analyse (50),
- Methanol zur Analyse (10),
- Ammoniaklösung 25% zur Analyse (10),
- demineralisiertes Wasser (10).

Das Verfahren wird so durchgeführt, dass auf die stationäre Phase (DC-Fertigfolie) 5 µl der verdünnten Prüflösung in der Bandbreite von 5 mm bandförmig aufgetragen wird. Nach einer ca. zehnminütigen Trocknungszeit wird die stationäre Phase in die mit Laufmittel befüllte Doppeltrogkammer gestellt. Die Chromatographie erfolgt nach den vorstehenden Parametern bei einer Temperatur von 20°C. Erreicht das Laufmittel (das Lösungsmittelgemisch) das Ende der Trennstrecke, die vorliegend 7 cm beträgt, wird die stationäre Phase aus der Kammer entnommen und an der Luft getrocknet. Im so erhaltenen Chromatogramm ist/sind die Zone(n) des/der Farbstoffe der Prüflösungen visuell erkennbar. Aus dem Verhältnis der Laufstrecke der Farbstoffe zur Gesamttrennstrecke (7 cm) kann der R_{F}-Wert berechnet werden.

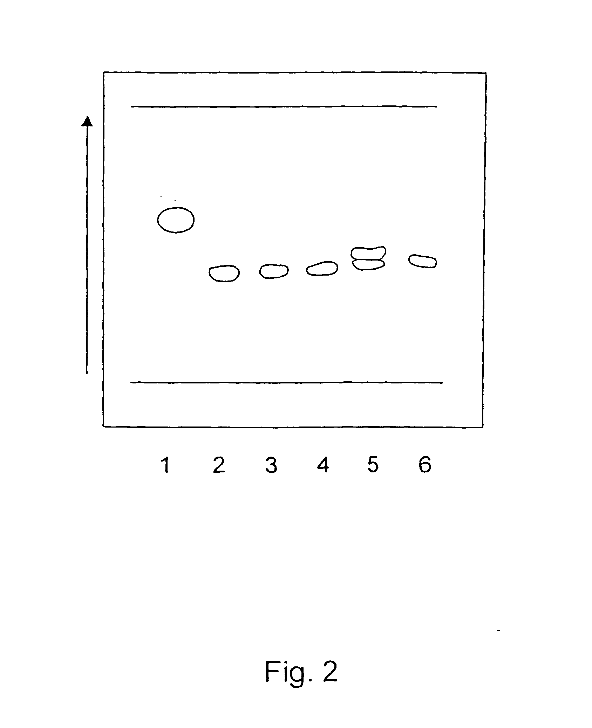

Figur 2 zeigt ein Chromatogramm eines erfindungsgemäßen Nässeindikators mit einem Azofarbstoff (1) sowie nicht erfindungsgemäßer Nässeindikatoren (2) - (6) auf Basis von Triphenylmethanfarbstoffen. Die Trennung erfolgt in der angedeuteten Pfeilrichtung zwischen der unteren Auftragszone (angedeutet durch eine waagrechte Linie) und der oberen Laufmittelfront (angedeutet durch die obere waagrechte Linie). Die nicht erfindungsgemäßen Nässeindikatoren (2) - (6) auf Basis von Triphenylmethanfarbstoffen zeigen alle einen geringeren R_{F}-Wert als 0,48. Dies bedeutet, dass die Wechselwirkungen zwischen dem Farbstoff der nicht erfindungsgemäßen Nässeindikatoren und der stark polaren Oberfläche der stationären Phase (Kieselgelplatte) derart ist, dass sie hieran stark haften und sich bei Einwirkung eines verhältnismäßig unpolaren Lösungsmittels hiervon schlecht ablösen lassen. Das erfindungsgemäße Nässeindikator-System auf Basis eines Azofarbstoffs liefert hingegen eine geringe Neigung zum Anhaften an der polaren Oberfläche der stationären Phase.

Es hat sich als vorteilhaft erwiesen, wenn der Farbstoff des Nässeindikators aus der Gruppe der Azofarbstoffe entstammt, die einen größeren R_{F}-Wert als 0,48 bestimmt nach der beschriebenen Methode aufweisen.

Für eine weitere Prüfung, ob die Zusammensetzung des Nässeindikators geeignet ist, bei Kontakt mit wässriger Flüssigkeit eine Ablösung oder Auflösung der visuell wahrnehmbaren strukturierten Anordnung bis zur Unkenntlichkeit zu gewährleisten, ist ein modifizierter "Run-off-Test", angelehnt an die Methode Edana - Run-off 152.1-02, vorgesehen, der nachfolgend beschrieben wird.

### Run-off-Test:

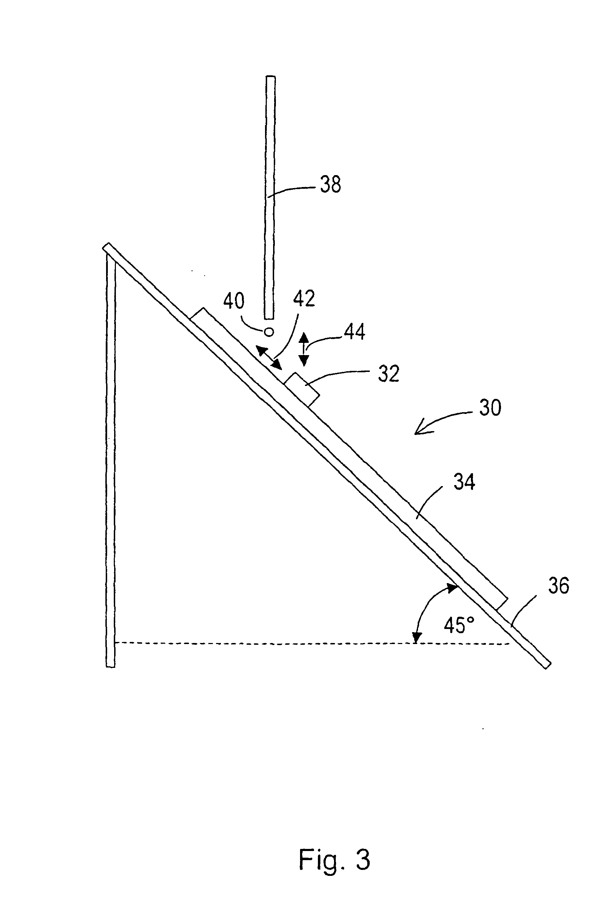

Mit diesem Test kann geprüft werden, ob die Zusammensetzung des Nässeindikators für die beabsichtigte Verwendung als wasserlöslicher Nässeindikator hinreichend ist. Hierzu wird 1 µl der zu testenden Tinte (des Nässeindikators) 32 auf das betreffende mikroporöse atmungsaktive Folienmaterial 34 des Rückenblatts aufgebracht und trocknen gelassen, so dass das. Lösungsmittel des Nässeindikators verdampfen kann. Anschließend wird ein Prüfling 30, welcher den Tintenfleck 32 umfasst, ausgestanzt und auf einer Trägerplatte mit einer schiefen Ebene 36, die gegen die Horizontale um 45° geneigt ist (s. Figur 3), fixiert, und zwar etwa 10 mm in Ebenenrichtung entfernt von einem oberhalb gelegenen Auftreffpunkt 42 für Flüssigkeit. An diesem Auftreffpunkt wird aus einer Bürette 38 aus einer Entfernung von 10 mm (44) oberhalb der Ebene des Prüflings 30 mit einer Tropfgeschwindigkeit von 1 Tropfen pro Sekunde wässrige Flüssigkeit 40, insbesondere 0,9% ige NaCl-Lösung auf die schiefe Ebene getropft, so dass diese Flüssigkeit über den Tintenfleck abläuft, so dass das Ablösen der Tinte visuell beobachtet werden kann und das hierfür erforderliche Flüssigkeitsvolumen notiert werden kann.

Nässeindikatoren, bzw. die Farbstoffe in den Nässeindikatoren, die bei der Durchführung der Dünnschichtchromatographie eine große Laufstrecke zurücklegen und/oder insbesondere auch bei dem Run-off-Test ohne weiteres abgewaschen werden, eignen sich a priori für die Aufbringung auf mikroporöse atmungsaktive Folien als Nässeindikator.

Erfindungsgemäß wurde festgestellt, dass es sich als vorteilhaft erweist, wenn der Farbstoff des Nässeindikators aus der Gruppe der Azofarbstoffe entstammt.

Die mikroporöse atmungsaktive Folie des Rückenblattes des absorbierenden Einmal-Artikels besteht vorzugsweise aus thermoplastischen Polymeren. Vorzugsweise besteht diese Folie aus Polyolefinen, insbesondere vorzugsweise aus Polyethylen und/oder Polypropylen. Hierbei werden LDPE (Low Density Polyethylen), LLDPE (Linear Low Density Polyethylen), MDPE (Medium Density Polyethylen) und/oder HDPE (High Densitiy Polyethylen) sowie analog verschiedene Polypropylene unterschiedlicher Dichte sowie Copolymerisate verwendet.

Insbesondere erweist sich ein Einmal-Artikel als vorteilhaft, bei dem die mikroporöse atmungsaktive Folie Calciumcarbonat als anorganischen Füllstoff enthält. Der Füllstoffanteil beträgt dabei in vorteilhafter Weise 30 bis 80 Gew.-%, insbesondere 40 bis 70 Gew.-% und weiter insbesondere 50 bis 60 Gew.-% bezogen auf die Masse der Folie.

Die mikroporösen, atmungsaktiven Folien haben inbesondere ein Flächengewicht von 8 g/m² bis 25 g/m², vorzugsweise von 10 g/m² bis 18 g/m².

Die mikroporösen, atmungsaktiven Folien weisen eine Atmungsaktivität gemessen als Wasserdampfdurchlässigkeit (WVTR) gemessen nach DIN 53 122-1 (Ausgabe: 2001-08) von mindestens 300 g/m²/24h, vorzugsweise von mindestens 500 g/m²/24h, weiter vorzugsweise von mindestens 1000 g/m²/24h auf.

Die mikroporösen, atmungsaktiven Folien weisen eine Wasserundurchlässigkeit auf, bestimmt als Wassersäule nach DIN EN 20811 (Ausgabe: Deutsche Fassung EN 20811:1992) von mindestens 30 cm, insbesondere von mindestens 50 cm, vorzugsweise von mindestens 100 cm, weiter vorzugsweise von mindestens 150 cm.

In Weiterbildung der Erfindung beträgt die durchschnittliche Porengröße der mikroporösen atmungsaktiven Folie des Rückenblattes höchstens 5 µm, insbesondere höchstens 3 µm, weiter insbesondere höchstens 1 µm und weiter insbesondere höchstens 0,5 µm. Die Ermittlung der Porengröße kann insbesondere mithilfe eines Rasterelektronenmikroskops vorgenommen werden. Dabei wird als Porengröße einer einzelnen Pore die größtmögliche lichte Weite einer auf der Auftragsseite der Folie erkennbaren Pore verstanden. Zur Berechnung der mittleren Porengröße werden die gemessenen Einzelwerte arithmetisch gemittelt.

Vorteilhafterweise ist die mikroporöse, atmungsaktive Folie, die üblicherweise in einem Reckungsprozess der Folie zur Erzeugung der Mikroporosität um die anorganischen Füllstoffe hergestellt ist, weiteren Behandlungsschritten, so wie in EP 0 768 168 B1 beschrieben, unterworfen worden, nämlich insbesondere der Aufheizung der Folienbahn auf vorzugweise mindestens 70 °C und einer anschließende vollflächigen Schockkühlung an einem Walzenpaar umfassend eine Gummiwalze und eine Prägewalze. Vorteilhafterweise ist die derart weiter bearbeitete Folie einer anschließenden zonenweisen Streckung über konturierte Walzenpaare unterzogen. Dieser Prozess ist auch als Ring-rolling-Prozess bekannt. Das Ring-rolling kann quer oder parallel zur Maschinenrichtung erfolgen.

Es erweist sich auch als vorteilhaft, wenn die mikroporöse atmungsaktive Folie auf der dem Saugkörper zugewandten Seite coronabehandelt ist. Insbesondere eignet sich eine Coronabehandlung, die zu einer Oberflächenspannung von 30 bis 40, insbesondere von 32 bis 38 und weiter insbesondere von 34 bis 36 dyn bei dem Folienmaterial führt. Durch eine Coronabehandlung wird eine Folie, insbesondere eine Polyolefinfolie, für einen Beschichtungsvorgang, insbesondere zum Bedrucken konditioniert. Dies eignet sich auch für das Aufbringen eines Nässeindikators der hier interessierenden Art, damit dieser einerseits gut haftend, andererseits aber ohne weiteres und sofort bei Flüssigkeitsbeaufschlagung ablösbar ist.

Im Hinblick auf eine geeignete Oberflächenkonditionierung erweist es sich auch als vorteilhaft, wenn die mikroporöse atmungsaktive Folie des Rückenblattes mit einer sehr feinen dreidimensionalen Oberflächenstruktur versehen ist. Diese Oberflächenstruktur kann vorteilhafterweise nach einem in EP 0 768 168 B1 beschriebenen Verfahren mit Hilfe einer Strukturwalze erzeugt werden.

In weiterer vorteilhafter Ausführung des absorbierenden Einmal-Artikels handelt es sich bei dem flüssigkeitsundurchlässigen Rückenblatt um ein textil anmutendes Rückenblatt (textile backsheet). Vorteilhafterweise ist die mikroporöse, atmungsaktive Folie auf der dem Nässeindikator abgewandten Seite der Folie mit einem Vlies laminiert. Vorzugsweise ist die Folie mit dem Vlies thermisch laminiert. Die thermische Laminierung kann nach den dem Fachmann bekannten Methoden erfolgen (Thermosiegeln, Air-through). Die Laminierung von Folie zu Vlies kann auf der Basis von Schmelzpunktverbindungen in einem angeordneten Muster erfolgen. Weiter vorzugsweise ist die Folie mit dem Vlies thermisch vollflächig laminiert.

Als Vlies kann jegliche Art von Vliesmaterial eingesetzt werden. Vorzugsweise werden kardierte Vliese eingesetzt. Weiter vorzugsweise werden spunbond-Materialien, also Spinnvliese, eingesetzt, insbesondere auf Basis von Polyolefinen aus der Gruppe Polypropylen und Polyethylen. Die eingesetzten Vliese haben ein Flächengewicht von 8 g/m² bis 25 g/m², vorzugsweise von 10 g/m² bis 20 g/m².

Das Rückenblatt aus dem Vlies-Folien-Laminat weist ein Gesamtflächengewicht von mindestens 23 g/m², vorzugsweise von mindestens 29 g/m², insbesondere bevorzugt von mindestens 34 g/m² auf.

Weitere Merkmale, Vorteile und Einzelheiten der Erfindung ergeben sich aus den beigefügten Patentansprüchen, und aus der zeichnerischen Darstellung und nachfolgenden Beschreibung der Erfindung.

In der Zeichnung zeigt:
- Figur 1: eine schematische Außenansicht eines erfindungsgemäßen absorbierenden Inkontinenzartikels mit einem Nässeindikator;
- Figur 2: eine schematische Darstellung eines Chromatogramms verschiedener Nässeindikator-Systeme;
- Figur 3: eine Darstellung der Anordnung zur Durchführung des Run-off-Tests;
- Figur 4 a und b: zwei Positionen einer Vorrichtung zur Durchführung des Abwischtests;
- Figur 5: das Ergebnis der Durchführung eines Abwischtests bei zwei verschiedenen Nässeindikator-Systemen und
- Figur 6: eine rasterelektronischenmikroskopische Darstellung der Oberfläche einer mikroporösen atmungsaktiven Folie des Rückenblatts.

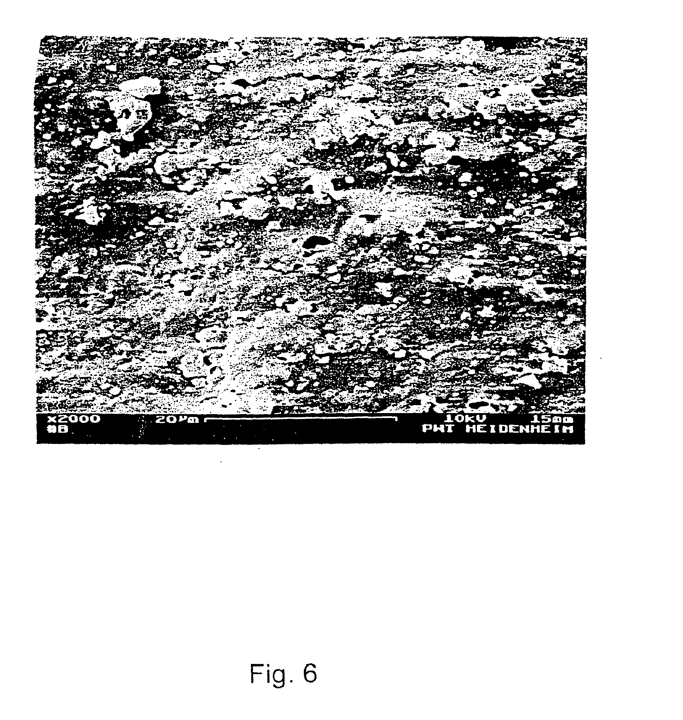

Figur 1 zeigt eine Draufsicht auf die körperabgewandte Außenseite eines erfindungsgemäßen Einmal-Artikels 2 in Form einer Inkontinenzwindel 3. Die Inkontinenzwindel 3 umfasst einen Haupt- oder Chassisteil 4 mit einem die äußere Sichtseite bildenden flüssigkeitsundurchlässigen, jedoch mikroporösen atmungsaktiven Rückenblatt 5. Die Mikroporosität des Rückenblatts 5 ist dabei durch Zugabe eines anorganischen Füllstoffs, insbesondere in Form von CaCO₃, zu einer Folie, insbesondere aus Polyolefin, und Recken der Folie des Rückenblatts 5 erreicht. Die Inkontinenzwindel umfasst des Weiteren ausladende Seitenklappen 6, um die Windel arm Körper eines Benutzers zu fixieren.
Auf der körperzugewandten Innenseite des Rückenblatts 5 ist eine strukturierte Anordnung 6 direkt aufgebracht. Die strukturierte Anordnung bildet einen visuell wahrnehmbaren direkt auf die innere Oberfläche der polymeren Folie aufgedruckten Nässeindikator 7, der in Kontakt mit Körperflüssigkeiten ablösbar ist. Die Zusammensetzung des Nässeindikators ist derart abgestimmt auf die im vorliegenden Fall polare Oberfläche der Folie auf Basis von Polyolefinen mit einem anorganischen Füllstoff, vorzugsweise CaCO₃, dass bei Flüssigkeitsbeaufschlagung sich der Nässeindikator 7 quasi sofort bis zur Unkenntlichkeit ablöst oder auflöst. Dies ist dann durch das vorzugsweise gut durchsichtige Material des Rückenblatts hindurch sofort visuell erkennbar. Eine rasterelektronenmikroskopische Darstellung der körperzugewandten Oberfläche des Rückenblatts 5 zeigt Figur 6. Die elektronenmikroskopische Aufnahme wurde bei einer Spannung von 10 kV durchgeführt und ist in einer 2000-fachen Vergrößerung mit einem eingeblendeten Maßstab von 20 µm dargestellt.

## Patentansprüche

1. Absorbierender Einmal-Artikel (2), umfassend einen Körperflüssigkeiten speichernden Saugkörper und ein zumindest abschnittsweise flüssigkeitsundurchlässiges Rückenblatt (5), wobei auf der dem Saugkörper zugewandten Seite der Folie ein Nässeindikator (7) in Form einer visuell wahrnehmbaren strukturierten Anordnung (6) vorgesehen ist, wobei sich die strukturierte Anordnung bei Kontakt mit wässriger Flüssigkeit bis zur Unkenntlichkeit ablöst, **dadurch gekennzeichnet, dass** das flüssigkeitsundurchlässige Rückenblatt (5) eine mikroporöse atmungsaktive Folie mit 30 - 80 Gew.-% CaCO₃ als anorganischer Füllstoff aufweist und dass der Nässeindikator (7) tintenbasiert ist und mit einer Schichtdicke von mindestens 2 µm direkt auf die mikroporöse Folie aufgebracht ist und dass der Nässeindikator (7) einen Azofarbstoff mit einem R_{F}-Wert größer 0,48 bestimmt nach der beschriebenen Methode umfasst.

2. Einmal-Artikel nach Anspruch 1, **dadurch gekennzeichnet, dass** der Nässeindikator (7) mit einer Schichtdicke von von mindestens 5 µm, weiter vorzugsweise von 24 µm auf die Folie aufgebracht ist.

3. Einmal-Artikel nach Anspruch 1 oder 2, **dadurch gekennzeichnet, dass** der Nässeindikator (7) mit einem Trockengewicht von mindestens 0,00024 g/cm², vorzugsweise von mindestens 0,00049 g/cm², weiter vorzugsweise von 0,0024 g/cm² auf die Folie aufgebracht ist.

4. Einmal-Artikel nach einem der vorstehenden Ansprüche, **dadurch gekennzeichnet, dass** die strukturierte Anordnung (6) des Nässeindikators (7) in Form von Symbolen, Kodierungen, Schriftzeichen aufgebracht ist.

5. Einmal-Artikel nach einem der vorstehenden Ansprüche, **dadurch gekennzeichnet, dass** die Folie aus einem Polymer besteht aus der Gruppe Polyethylen und Polypropylen.

6. Einmal-Artikel nach einem der vorstehenden Ansprüche, **dadurch gekennzeichnet, dass** Calciumcarbonat in einem Anteil von 40 - 70 Gew.-%, weiter vorzugsweise von 50 - 60 Gew.-% bezogen auf die Masse der Folie enthalten ist.

7. Einmal-Artikel nach einem der vorstehenden Ansprüche, **dadurch gekennzeichnet, dass** die Folie auf der Auftragsseite des Nässeindikators coronabehandelt ist.

8. Einmal-Artikel nach einem der vorstehenden Ansprüche, **dadurch gekennzeichnet, dass** die Folie eine Oberflächenstruktur, insbesondere eine Oberflächenprägung aufweist.

9. Einmal-Artikel nach einem der vorstehenden Ansprüche, **dadurch gekennzeichnet, dass** die dem Nässeindikator gegenüberliegende Seite der Folie mit einem Vlies laminiert ist.

10. Einmal-Artikel nach Anspruch 9, **dadurch gekennzeichnet, dass** Folie mit dem Vlies thermisch laminiert ist.

11. Einmal-Artikel nach Anspruch 9 oder 10, **dadurch gekennzeichnet, dass** die Folie mit dem Vlies vollflächig thermisch laminiert ist.

## Claims

1. An absorbent single-use article (2) comprising an absorbent element for storing bodily fluids and a backsheet (5) which is fluid impermeable at least in sections, wherein on the side of the film facing the absorbent element, a moisture indicator (7) is provided in the form of a visually perceptible textured arrangement (6), the textured arrangement being detached beyond recognition upon contact with aqueous fluid, **characterized in that** the fluid impermeable backsheet (5) comprises a microporous breathable film with 30 - 80 weight percent CaCO₃ as an inorganic filler material and that the moisture indicator (7) is ink-based and is directly applied to the microporous film with a coating thickness of at least 2 µm and that the moisture indicator (7) comprises an azo dye with an R_{F} -value greater than 0.48 determined by the method described.

2. A single-use article (2) according to claim 1, **characterized in that** the moisture indicator (7) is applied to the film with a coating thickness of at least 5 µm and more preferably of at least 24 µm.

3. A single-use article (2) according to claim 1 or 2, **characterized in that** the moisture indicator (7) is applied to the film with a dry weight of at least 0.00024 g/cm² , preferably of at least 0.00049 g/cm² and more preferably of at least 0.0024 g/cm².

4. A single-use article (2) according to one of the preceding claims, **characterized in that** the textured arrangement (6) of the moisture indicator (7) is applied in the form of symbols, codes and characters.

5. A single-use article (2) after one of the preceding claims, **characterized in that** the film consists of a polymer selected from the group of polyethylenes and polypropylenes.

6. A single-use article (2) according to one of the preceding claims, **characterized in that** calcium carbonate is contained in an amount of 40 - 70 % by weight relative to the mass of the film and more preferably by 50 - 60 % by weight relative to the mass of the film.

7. A single-use article (2) according to one of the preceding claims, **characterized in that** the film is corona treated on an application side of the moisture indicator.

8. A single-use article (2) according to one of the preceding claims, **characterized in that** the film has a surface texture, particularly a surface stamping.

9. A single-use article (2) according to one of the preceding claims, **characterized in that** the side of the film lying opposite to the moisture indicator is laminated with a non-woven material.

10. A single-use article (2) according to claim 9, **characterized in that** the film is thermally laminated with the non-woven material.

11. A single-use article (2) according to claim 9 or 10, **characterized in that** the film is thermally laminated with the non-woven material over its entire surface.

## Revendications

1. Article absorbant à usage unique (2) comprenant un corps absorbant retenant les fluides corporels et une feuille arrière (5) imperméable aux liquides au moins par endroits, un indicateur d'humidité (7) étant prévu sous la forme d'un agencement structuré visuellement perceptible (6) sur le côté de la feuille orienté vers le corps absorbant, l'agencement structuré s'effaçant jusqu'à être méconnaissable au contact d'un fluide aqueux, **caractérisé en ce que** la feuille arrière (5) imperméable aux liquides présente une feuille respirante microporeuse comprenant de 30 à 80 % en poids de CACO₃ comme matière de charge inorganique, **en ce que** l'indicateur d'humidité (7) est à base d'encre et est appliqué directement sur la feuille microporeuse avec une épaisseur de couche d'au moins 2 µm, et **en ce que** l'indicateur d'humidité (7) comprend un colorant azoïque avec une valeur R_{F} supérieure à 0,48 calculée suivant la méthode décrite.

2. Article à usage unique selon la revendication 1, **caractérisé en ce que** l'indicateur d'humidité (7) est appliqué sur la feuille avec une épaisseur de couche d'au moins 5 µm, de façon davantage préférée de 24 µm.

3. Article à usage unique selon la revendication 1 ou 2, **caractérisé en ce que** l'indicateur d'humidité (7) est appliqué sur la feuille avec un poids à sec d'au moins 0,00024 g/cm², de préférence d'au moins 0,00049 g/cm², de façon davantage préférée de 0,0024 g/cm².

4. Article à usage unique selon l'une des revendications précédentes, **caractérisé en ce que** l'agencement structuré (6) de l'indicateur d'humidité (7) est appliqué sous la forme de symboles, de codages, de caractères.

5. Article à usage unique selon l'une des revendications précédentes, **caractérisé en ce que** la feuille est composée d'un polymère issu du groupe des polyéthylènes et des polypropylènes.

6. Article à usage unique selon l'une des revendications précédentes, **caractérisé en ce que** du carbonate de calcium est contenu dans une part de 40 à 70 % en poids, de façon davantage préférée de 50 à 60 % en poids rapporté à la masse de la feuille.

7. Article à usage unique selon l'une des revendications précédentes, **caractérisé en ce que** la feuille est traitée au corona sur le côté d'application de l'indicateur d'humidité.

8. Article à usage unique selon l'une des revendications précédentes, **caractérisé en ce que** la feuille présente une structure de surface, en particulier un gaufrage de surface.

9. Article à usage unique selon l'une des revendications précédentes, **caractérisé en ce que** le côté opposé à l'indicateur d'humidité de la feuille est stratifié avec un non-tissé.

10. Article à usage unique selon la revendication 9, **caractérisé en ce que** la feuille est stratifiée thermiquement avec un non-tissé.

11. Article à usage unique selon la revendication 9 ou 10, **caractérisé en ce que** la feuille est stratifiée thermiquement sur toute sa surface avec le non-tissé.
